# EUROPÄISCHE PATENTSCHRIFT

(11) **EP 0 507 300 B1**
(45) Veröffentlichungstag und Bekanntmachung des Hinweises auf die Patenterteilung: **25.01.1995**
(21) Anmeldenummer: 92105696.6
(22) Anmeldetag: 02.04.1992
(51) Int. Cl.: C07C 5/32

(54) **Verfahren zur katalytischen Dehydrierung von Kohlenstoff-Verbindungen**
Process for the catalytic dehydrogenation of carbon compounds
Procédé de déshydrogénation catalytique de composés carbonés

(30) Priorität: 05.04.1991 DE 4111071
(43) Veröffentlichungstag der Anmeldung: 07.10.1992
(73) Patentinhaber: Linde Aktiengesellschaft, 65189 Wiesbaden (DE); BASF Aktiengesellschaft, 67063 Ludwigshafen (DE)
(72) Erfinder: Fritz, Peter Matthias, Dr. rer. nat., W-8000 München 90 (DE); Walser, Franz, Dipl.-Ing. (FH), W-8110 Murnau (DE); Bölt, Heinz, Dipl.-Ing. (FH), W-8037 Olching (DE); Stabel, Uwe, Dr.-Ing., W-6803 Edingen/Neckarhausen (DE); Wunsch, Gerd, Dr.-Ing., W-6720 Speyer (DE)
(74) Vertreter: Schaefer, Gerhard, Dr.

(56) Entgegenhaltungen:
- US-A- 3 708 550
- US-A- 3 773 850
- US-A- 4 052 338
- US-A- 4 613 722

## Beschreibung

Die Erfindung betrifft ein Verfahren zur katalytischen Dehydrierung von Kohlenstoff-Verbindungen, insbesondere von Propan und/oder Butan, wobei während der Dehydrierung Kohlenstoff freigesetzt wird und bei der Dehydrierung ein mit Kohlenstoff reagierender Zusatzstoff zugegeben wird.

Aus der DE-PS 34 39 176 ist bekannt, daß bei der katalytischen Dehydrierung von Butan Kohlenstoff freigesetzt wird. Im Dehydrierungsprozeß lagern sich auf dem Katalysator Kohlenstoffpartikel (Koks) ab, was zu einer Desaktivierung des Katalysators führt. Der Katalysator muß daher nach einer Laufzeit von etwa 6 bis 8 Stunden regeneriert werden, indem die Kohlenstoff-Ablagerungen auf dem Katalysator mit einem oxidierenden Medium abgebrannt werden. Da der Katalysator in regelmäßigen Abständen regeneriert werden muß, ist es erforderlich, den Reaktor in zwei Teilreaktionsräume zu unterteilen oder zwei Reaktoren einzusetzen, um in einem Teilreaktionsraum bzw. in einem Reaktor zu dehydrieren, während im anderen Teilreaktionsraum bzw. im zweiten Reaktor die Regenerierung des Katalysators durchgeführt wird. Dieser Wechselbetrieb bringt einen erheblichen apparativen Aufwand mit sich.

Aus der US-A-3 708 550 ist ein Verfahren zur katalytischen Dehydrierung von Olefinen in Diolefine bekannt, wobei sich bei der Dehydrierung harte Kohlenstoff-haltige Ablagerungen bilden. Diese können dadurch vollständig beseitigt werden, daß kontinuierlich oder zeitweise ein schwefelhaltiges Additiv in die Reaktionszone eingespeist wird. Als schwefelhaltige Additive sind dabei H₂S (bevorzugt), Organomercaptane oder Organosulfide genannt.

Der vorliegenden Erfindung liegt daher die Aufgabe zugrunde, ein Verfahren der eingangs genannten Art aufzuzeigen, welches die Koksablagerungen auf dem Katalysator reduziert und bei welchem der Zusatzstoff nicht zu Nachteieln im Gesamtprozess führt.

Diese Aufgabe wird erfindungsgemäß dadurch gelöst, daß CO₂, O₂, H₂ oder Gemische dieser Stoffe als Zusatzstoff zugegeben werden.

Der Zusatzstoff reagiert mit dem bei der Dehydrierung freigesetzten Kohlenstoff und bindet ihn.

Durch die erfindungsgemäße Zugabe beispielsweise von Kohlendioxid in den Dehydrierreaktor wird Kohlenstoff nach der Vergasungsreaktion

C + CO₂ ----------> 2 CO

im Kohlenmonoxid gebunden. Durch die Zugabe des Zusatzstoffes werden die Koksablagerungen auf dem Katalysator wesentlich verringert bzw. vermieden. Eine Desaktivierung des Katalysators durch Koks wird somit verhindert. Damit lassen sich zumindest die Laufzeiten des Katalysators bis zur Regeneration wesentlich erhöhen.

Das erfindungsgemäße Verfahren ist bei allen katalytischen Dehydrierprozessen anwendbar, bei denen Kohlenstoff freigesetzt wird und den Katalysator desaktiviert. Beispiele für derartige Dehydrierprozesse stellen die Dehydrierung von Propan, i-Butan, n-Butan, Ethylbenzol oder Gemischen hieraus dar.

Die Zusatzstoff-Einspeisung kann periodisch erfolgen, d.h. der Zusatzstoff wird pulsartig in den Dehydrierreaktor eingeleitet. Besondere Vorteile ergeben sich jedoch beim erfindungsgemäßen Verfahren dann, wenn der Zusatzstoff mit einem konstanten Volumenstrom, also kontinuierlich, während der Dehydrierung zugegeben wird.

Mit Vorteil wird der Zusatzstoff in den Reaktor zu Dehydrierung mit einer Menge von 1 vol.-ppm bis 50 vol.-%, bevorzugt von 1 vol.-ppm bis 10 vol.-%, eingespeist, wobei die angegebenen Mengen auf die zu dehydrierende Rohgasmenge bezogen sind.

In Weiterbildung des Erfindungsgedankens wird die Dehydrierung in einem Reaktor kontinuierlich betrieben, was bedeutet, daß das zu dehydrierende Rohgas und der erfindungsgemäß zugegebene Zusatzstoff jeweils mit einem bestimmten Volumenstrom unter einem konstanten Verhältnis in den Reaktor eingespeist werden.

Erfindungsgemäß findet die Dehydrierung mit besonderem Vorteil in einem einzigen Reaktor mit einem Reaktionsraum statt, ohne daß ein periodisches Umschalten im Dehydrierungsprozeß in verschiedene Teilreaktionsräume oder in verschiedene Reaktoren erfolgt, wie es nach dem oben zitierten Stand der Technik erforderlich ist.

Eine derartige Verfahrensführung erlaubt den Wegfall des Regeneriersystems. Vor allem erübrigt sich dadurch der Einbau von heißen Schaltventilen. Das bedeutet eine wesentliche Verringerung des apparativen Aufwandes und damit der Kosten. Gleichzeitig wird die Betriebssicherheit im Dehydrierungsprozeß erhöht.

Im folgenden wird die Erfindung anhand eines Ausführungsbeispiels näher erläutert:
Zur Dehydrierung von Propan wurden konstant während des ganzen Versuches 20 Nl/h Propan einem Dehydrierreaktor aufgegeben. Für den Versuch wurde, um ein unverfälschtes Ergebnis zu erhalten, nicht, wie bei industriellem Einsatz üblich, die Temperatur während des Dehydrierprozesses erhöht, sondern der Dehydrierreaktor wurde isotherm gefahren. Die Temperatur im Dehydrierreaktor betrug im Versuch 550°C. Zunächst wurde die Dehydrierung des Propans ohne Zusatzstoff-Zugabe durchgeführt. Hierbei wurde eine Dehydrieraktivität, d.h. C₃-Conversion, von 30% zu Beginn, von 25% nach 6 h, von 20% nach 12 h und von 15% nach 18 h gemessen. Bei der erfindungsgemäßen Zugabe an CO₂ von 16 vol.-ppm in den Dehydrierreaktor ergab sich eine Dehydrieraktivität bzw. eine C₃-Konversion über den gesamten Zeitraum von 23 bis 24%. Damit war zwar die Dehydrieraktivität bei der erfindungsgemäßen CO₂-Zugabe am Anfang etwas geringer. Sie lag aber nach wenigen Stunden über der Dehydrieraktivität ohne CO₂-Zugabe.

## Patentansprüche

1. Verfahren zur katalytischen Dehydrierung von Kohlenstoff-Verbindungen, insbesondere von Propan und/oder Butan, wobei während der Dehydrierung Kohlenstoff freigesetzt wird und bei der Dehydrierung ein mit Kohlenstoff reagierender Zusatzstoff zugegeben wird, **dadurch gekennzeichnet**, daß CO₂, O₂, H₂ oder Gemische dieser Stoffe als Zusatzstoff zugegeben werden.

2. Verfahren nach Anspruch 1, dadurch gekennzeichnet, daß der Zusatzstoff mit konstantem Durchsatz zugegeben wird.

3. Verfahren nach einem der Ansprüche 1 oder 2, dadurch gekennzeichnet, daß eine Zugabe des Zusatzstoffes bezogen auf das zu dehydrierende Rohgas von 1 vol.-ppm bis 50 vol.-%, bevorzugt von 1 vol.-ppm bis 10 vol.-%, erfolgt.

4. Verfahren nach einem der Ansprüche 1 bis 3, dadurch gekennzeichnet, daß bei der Dehydrierung ein Reaktor kontinuierlich betrieben wird.

5. Verfahren nach einem der Ansprüche 1 bis 4, dadurch gekennzeichnet, daß die Dehydrierung in einem Reaktor mit einem Reaktionsraum durchgeführt wird.

## Claims

1. Process for catalytic dehydrogenation of carbon compounds, in particular of propane and/or butane, in which carbon is liberated during the dehydrogenation and an additive reacting with carbon is added during the dehydrogenation, characterised in that CO₂, O₂, H₂ or mixtures of these substances are added as additive.

2. Process according to claim 1, characterised in that the additive is added at a constant throughput.

3. Process according to one or claims 1 or 2, characterised in that from 1 ppm vol. to 50% vol., preferably from 1 ppm vol. to 10% vol., of additive is added in relation to the untreated gas to be dehydrogenated.

4. Process according to one of claims 1 to 3, characterised in that one reactor is operated continuously during the dehydrogenation.

5. Process according to one of claims 1 to 4, characterised in that the dehydrogenation is carried out in one reactor with one reaction chamber.

## Revendications

1. Procédé de déshydrogénation catalytique de composés carbonés, notamment de propane et/ou de butane, dans lequel pendant la déshydrogénation sont libérés des composés carbonés et est ajouté un adjuvant réagissant avec les composés carbonés, ce procédé étant caractérisé en ce qu'en tant qu'adjuvant est employé CO₂, O₂, H₂ ou un de leurs mélanges.

2. Procédé selon la revendication 1, caractérisé en ce qu'un débit constant d'adjuvant est ajouté.

3. Procédé selon une des revendications 1 ou 2, caractérisé en ce que les proportions d'adjuvant ajouté par rapport au gaz brut traité sont de 1 vol.-ppm à 50 vol.-% et, de préférence de 1 vol.-ppm à 10 vol.-%.

4. Procédé selon une des revendications 1 à 3, caractérisé en ce qu'un réacteur fonctionne en continu durant la déshydrogénation.

5. Procédé selon la revendication 1 à 4, caractérisé en ce que la déshydrogénation est mise en oeuvre dans un réacteur muni d'une enceinte de réaction.
